# EUROPEAN PATENT SPECIFICATION

(11) **EP 0 385 916 B1**
(45) Date of publication and mention of the grant of the patent: **12.01.1994**
(21) Application number: 90630047.0
(22) Date of filing: 22.02.1990
(51) Int. Cl.: A61M 5/155

(54) **Device for dispensing a liquid particularly useful for delivering medicaments at a predetermined rate.**
Vorrichtung zur Abgabe einer Flüssigkeit, insbesondere zum Gebrauch für die Ausgabe von Medikamenten in einer vorherbestimmten Menge.
Appareil pour délivrer un liquide particulièrement utile pour administrer des médicaments à un débit prédéterminé.

(30) Priority: 24.02.1989 IL 89400; 30.06.1989 IL 90816
(43) Date of publication of application: 05.09.1990
(73) Proprietor: S.I. SCIENTIFIC INNOVATIONS LTD., Tel Aviv 66033 (IL)
(72) Inventor: Gross, Joseph, Mazor 205 (IL); Zucker, Shlomo, Yavne (IL)
(74) Representative: Schmitz, Jean-Marie

(56) References cited:
- EP-A- 0 209 644
- WO-A-88/00065
- FR-A- 2 195 461
- US-A- 3 115 280
- US-A- 4 351 335

## Description

The present invention relates to a device for dispensing a liquid. The invention is particularly useful for delivering medicaments at predetermined rates, and is therefore described below with respect to this application.

There are many applications requiring the dispensing or delivering of a liquid at predetermined or precisely controlled rates. One of the applications requiring particularly precise rates of delivery is in systems for administering insulin or other medicaments, and very precise pumps have been devised for this purpose. However, such pumps are expensive to produce and maintain, and are inconvenient to refill with the periodic dosage requirements.

Various types of electrolytic or electrochemical pumps have been proposed for dispensing the liquid at a predetermined rate as controlled by the electrical current supplied to the electrolytic or electrochemical pump. US-A-3115280, EP-A-0 209 644 and WO-A-8 800 065.

The latter publication discloses a device for dispensing a liquid at a predetermined rate, comprising a container for receiving a supply of the liquid to be dispensed; means for subjecting the liquid to a pressurized gas to force liquid from the container, said means comprising an electrolytic cell having electrodes and an electrolyte capable of generating the pressurized gas in accordance with the rate of energization of the electrodes; and a piston assembly movable within the container and dividing it into a first expansible-contractible chamber for receiving the liquid to be dispensed, and a second expansible-contractible chamber for receiving the pressurized gas to dispense liquid from the first chamber of the container. Such a device will hereinafter be referred to as a device of the kind specified.

An object of the present invention is to provide a device of the kind specified but including protection against inadvertent mixing of the gas from the pressurized, second expansible-contractible chamber with the liquid to be dispensed from the first expansible-contractible chamber, e.g., because of an imperfect seal in the piston assembly between the two chambers.

According to the present invention, there is provided a device of the kind specified, characterized in that the piston assembly comprises a pair of pistons spaced apart by a connecting stem, and in that the container is formed with a vent opening in the space between said pistons to provide protection against inadvertent mixing of the gas from the second chamber with the liquid in the first chamber.

According to further features in the preferred embodiments of the invention described below, the second expansible-contractible chamber includes an electrolytic cell having electrodes adapted to be electrically energized, and an electrolyte capable of generating, upon the energization of the electrodes, the gas to thereby dispense the liquid from the container in accordance with the rate of energization of the electrodes.

The electrolyte of the electrolytic cell may be of any appropriate material, preferably of liquid or gel form, which generates a gas upon the energization of the electrodes and in accordance with the rate of energization of the electrodes. Examples of electrolytes which may be used include saline solution or other polar solutions or gels generating hydrogen, oxygen, nitrogen or carbon dioxide.

Further features and advantages of the invention will be apparent from the description below.

The invention is herein described, by way of example only, with reference to the accompanying drawings, wherein:
Fig. 1 is a longitudinal sectional view of the pumping unit in one form of medicament delivery device constructed in accordance with the present invention; and
Fig. 2 illustrates a complete medicament delivery device including the pumping unit of Fig. 1.

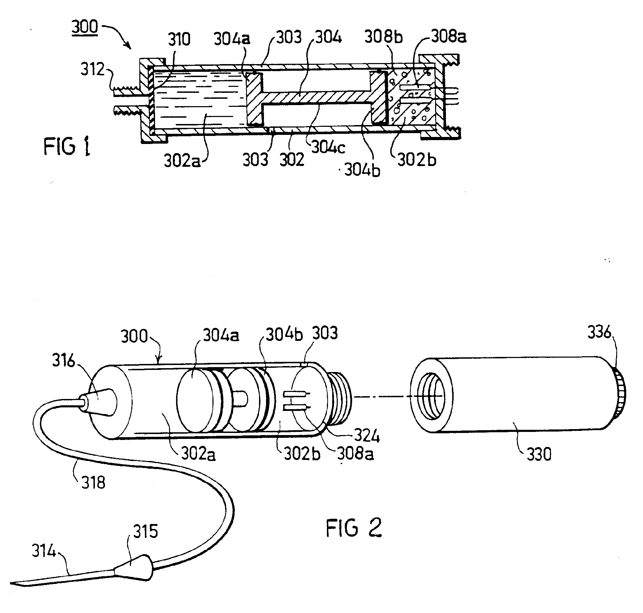

The medicament delivery device illustrated in Fig. 1, generally designated 300, includes a piston assembly 304 serving as a displaceable partition member within a container 302 dividing it into a drug-receiving chamber 302a and an electrolytic cell chamber 302b. Piston assembly 304 includes two pistons 304a and 304b spaced apart by an axially extending stem 304c joining the two pistons. In addition, container 302 is formed with one or more vent openings 303 in the space between the two pistons 304a, 304b. This construction isolates the drug in chamber 302a from the electrolyte 308b in chamber 302b, and also from the gas generated within that chamber by the energization of the electrodes 308a, since such electrolyte or gas, if it does penetrate past piston 304b, will be vented to the atmosphere via vent openings 303.

Fig. 2 illustrates a complete medicament delivery device utilizing the double-piston construction of Fig. 1. Thus, the syringe needle 314, including its fitting 315, is adapted to be attached to fitting 316 closing the end of the drug-receiving chamber 302a. The syringe needle 314 is connected to fitting 316 via another flexible tube 318, the latter tube including a piercing element for piercing a sealing membrane 310, Fig. 1, to permit the delivery of the drug from chamber 302a to the syringe needle 314.

Fig. 2 also illustrates a power supply unit 330 attachable to the fitting 324 at the opposite end of container 302 for energizing the electrodes within the electrolytic cell compartment 302b which generate the propelling gas. The power supply unit 330 includes a battery for energizing the electrodes 308a, a control circuit for controlling the energization of the electrodes, and a presetting knob 336 for presetting the rate of energization of the electrodes, and thereby the rate of delivery of the drug from compartment 302a.

## Claims

1. A device for dispensing a liquid at a predetermined rate, comprising: a container (302) for receiving a supply of the liquid to be dispensed; means (308a, 308b) for subjecting the liquid to a pressurized gas to force liquid from the container said means comprising an electrolytic cell (302 b) having electrodes (308 a) and an electrolyte (308 b) capable of generating the pressurized gas in accordance with the rate of energization of the electrodes; and a piston assembly (304) movable within said container and dividing same into a first expansible-contractible chamber (302a) for receiving the liquid to be dispensed, and a second expansible-contractible chamber (302b) for receiving the pressurized gas to dispense liquid from said first chamber of the container; characterized in that said piston assembly (304) comprises a pair of pistons (304a, 304b) spaced apart by a connecting stem (304c), and in that said container (302) is formed with a vent opening (303) in the space between said pistons to provide protection against inadvertent mixing of the gas from said second chamber with the liquid in said first chamber.

2. The device according to Claim 1, wherein said second expansible-contractible chamber (302b) includes an electrolytic cell having electrodes (308a) adapted to be electrically energized, and an electrolyte (308b) capable of generating, upon the energization of the electrodes, the gas to thereby dispense the liquid from the container in accordance with the rate of energization of said electrodes.

3. The device according to Claim 2, wherein the end of said container through which the liquid is to be dispensed is closed by a membrane (310) pierceable by a needle to permit dispensing the liquid.

4. The device according to any one of Claims 1-3, further including an electrical power supply (330) for energizing said electrodes, said electrical power supply being housed within a separate power supply unit attachable to said container.

5. The device according to Claim 4, wherein said power supply unit (330) includes a battery and an electrical control circuit for controlling the rate of energization of the electrodes, and thereby the rate of dispensing the liquid from the container.

6. The device according to Claim 5, wherein said electrical control circuit includes presettable means (336) for presetting the rate of energization of the electrodes.

## Patentansprüche

1. Vorrichtung zum Abgeben einer Flüssigkeit mit einer vorbestimmten Geschwindigkeit, mit: einem Behälter (302) zum Aufnehmen eines Vorrats der abzugebenden Flüssigkeit; einer Einrichtung (308a, 308b) zum Beaufschlagen der Flüssigkeit mit einem unter Druck stehenden Gas, um die Flüssigkeit aus dem Behälter hinauszudrücken, wobei die Einrichtung eine elektrolytische Zelle (302b) aufweist, die Elektroden (308a) und einen Elektrolyten (308b) hat, welche in der Lage sind, das unter Druck stehende Gas gemäß der Stärke der Stromversorgung der Elektroden zu erzeugen; und einer Kolbenbaugruppe (304), die in dem Behälter bewegbar ist und denselben in eine erste expandierbare-kontraktierbare Kammer (302a) zum Aufnehmen der abzugebenden Flüssigkeit und in eine zweite expandierbare-kontraktierbare Kammer (302b) zum Aufnehmen des unter Druck stehenden Gases zum Abgeben der Flüssigkeit aus der ersten Kammer des Behälters unterteilt; dadurch gekennzeichnet, daß die Kolbenbaugruppe (304) ein Paar Kolben (304a, 304b) aufweist, die durch einen Verbindungsschaft (304c) beabstandet sind, und daß der Behälter (302) mit einer Belüftungsöffnung (303) in dem Zwischenraum zwischen den Kolben versehen ist, um einen Schutz gegen unbeabsichtigtes Vermischen des Gases aus der zweiten mit der Flüssigkeit in der ersten Kammer zu bieten.

2. Vorrichtung nach Anspruch 1, wobei die zweite expandierbare-kontraktierbare Kammer (302b) eine elektrolytische Zelle enthält, die Elektroden (308a) hat, welche so ausgebildet sind, daß sie mit elektrischem Strom versorgbar sind, und einen Elektrolyten (308b), der in der Lage ist, bei der Stromversorgung der Elektroden das Gas zu erzeugen, um dadurch die Flüssigkeit aus dem Behälter gemäß der Stärke der Stromversorgung der Elektroden abzugeben.

3. Vorrichtung nach Anspruch 2, wobei das Ende des Behälters, über das die Flüssigkeit abgegeben werden soll, durch eine Membran (310) verschlossen ist, die mittels einer Nadel durchstechbar ist, um das Abgeben der Flüssigkeit zu gestatten.

4. Vorrichtung nach einem der Ansprüche 1-3, weiter mit einer elektrischen Stromversorgung (330) zum Versorgen der Elektroden mit Strom, wobei die elektrische Stromversorgung in einer separaten Stromversorgungseinheit untergebracht ist, die an dem Behälter befestigbar ist.

5. Vorrichtung nach Anspruch 4, wobei die Stromversorgungseinheit (330) eine Batterie und eine elektrische Steuerschaltung zum Steuern der Stärke der Stromversorgung der Elektroden und dadurch der Geschwindigkeit des Abgebens der Flüssigkeit aus dem Behälter aufweist.

6. Vorrichtung nach Anspruch 5, wobei die elektrische Steuerschaltung eine voreinstellbare Einrichtung (336) zum Voreinstellen der Stärke der Stromversorgung der Elektroden aufweist.

## Revendications

1. Dispositif pour distribuer un liquide à un débit prédéterminé, comprenant : un récipient (302) pour recevoir un approvisionnement du liquide à distribuer; des moyens (308a, 308b) pour soumettre le liquide à un gaz sous pression dans le but de forcer le liquide hors du récipient, lesdits moyens comprenant une pile électrolytique (302b) possédant des électrodes (308a) et un électrolyte (308b) capable de générer le gaz sous pression conformément au taux d'excitation des électrodes; ainsi qu'un assemblage de pistons (304) mobile au sein dudit récipient et subdivisant ce dernier en une première chambre (302a) apte à se dilater et à se contracter pour recevoir le liquide à distribuer, et une seconde chambre (302b) apte à se dilater et à se contracter pour recevoir le gaz sous pression destiné à distribuer le liquide depuis ladite première chambre du récipient; caractérisé en ce que ledit assemblage de pistons (304) comprend une paire de pistons (304a, 304b) espacés l'un de l'autre par une tige de liaison (304c) et en ce qu'une ouverture d'évent (303) est pratiquée dans ledit récipient (302) dans l'espace ménagé entre lesdits pistons pour procurer une protection contre un mélange se produisant par inadvertance entre le gaz provenant de la seconde chambre et le liquide dans ladite première chambre.

2. Dispositif selon la revendication 1, dans lequel ladite seconde chambre (302b) apte à se dilater et se contracter englobe une pile électrolytique possédant des électrodes (308a) conçues pour être excitées électriquement, ainsi qu'un électrolyte (308b) capable de générer lors de l'excitation des électrodes le gaz pour ainsi distribuer le liquide hors du récipient conformément au taux d'excitation desdites électrodes.

3. Dispositif selon la revendication 2, dans lequel l'extrémité dudit récipient à travers laquelle le liquide doit être distribué est fermé par une membrane (310) qui peut être percée par une aiguille pour permettre la distribution du liquide.

4. Dispositif selon l'une quelconque des revendications 1 à 3, englobant en outre une alimentation en énergie électrique (330) pour exciter lesdites électrodes, ladite alimentation en énergie électrique étant logée à l'intérieur d'une unité séparée d'alimentation en énergie qui peut être fixée audit récipient.

5. Dispositif selon la revendication 4, dans lequel ladite unité d'alimentation en énergie (330) englobe une batterie et un circuit de commande électrique pour commander le taux d'excitation des électrodes et ainsi le débit de distribution du liquide hors du récipient.

6. Dispositif selon la revendication 5, dans lequel ledit circuit de commande électrique englobe des moyens préréglables (306) pour prérégler le taux d'excitation des électrodes.
